Europäisches Patentamt

European Patent Office

Office européen des brevets

(11) Publication number: 0 593 828 A1

# EUROPEAN PATENT APPLICATION

(21) Application number: 92309720.8

(51) Int. Cl.5: C07C 2/66

(22) Date of filing: 23.10.92

(43) Date of publication of application:
27.04.94 Bulletin 94/17

(84) Designated Contracting States:
BE DE FR GB IT NL

(71) Applicant: MOBIL OIL CORPORATION
3225 Gallows Road
Fairfax, Virginia 22037-0001(US)

(72) Inventor: Le, Ouang Ngoc
330 Rhode Island Avenue
Cherry Hill, New Jersey 08002(US)
Inventor: Shim, Joosup
6 South Marion Avenue
Wenonah, New Jersey 08090(US)

(74) Representative: Colmer, Stephen Gary
Patent Department
c/o Mobil Services Company Limited
Mobil Court
3 Clements Inn
London WC2A 2EB (GB)

(54) Process for preparing alkyl-substituted aromatic compounds.

(57) In a process for preparing an alkyl-substituted aromatic compound, particularly an alkyl-substituted naphthalene with a high proportion of the alpha isomer, the aromatic compound is reacted with an alkylating agent having at least six carbon atoms at a temperature of 50°C to 300°C in the presence of an alkylation catalyst comprising a porous crystalline zeolite, particularly zeolite beta or USY, having an alpha value of less than 300.

EP 0 593 828 A1

This invention is directed to a process for preparing alkyl-substituted aromatic compounds and in particular alkyl-substituted naphthalenes with an increased ratio of alpha isomers to beta isomers.

It is known from, for example, US Patent No. 5026941 that alkylation of naphthalene with propylene in the presence of zeolite Y, which has been acid treated to increase its silica/alumina ratio to 10-350, and a saturated polycarboxylic acid is highly selective to the production of the beta isomer.

According to the present invention, it has now been found that by using certain low acidity zeolites (e.g., USY and zeolite beta) and low alkylation temperatures, naphthalenes can be alkylated with increased selectivity to the alpha isomer. The resultant alkylated naphthalene fluids exhibit improved thermal and oxidative stabilities when used as synthetic lubricants.

Thus the present invention resides in a process for preparing an alkyl-substituted aromatic compound comprising reacting an aromatic compound with an alkylating agent possessing an alkylating aliphatic group having at least six carbon atoms at a temperature of 50°C to 300°C in the presence of an alkylation catalyst comprising a porous crystalline zeolite having an alpha value of less than 300 to form an alkylated aromatic compound possessing at least one alkyl group derived from the alkylating agent.

The starting materials for use in the present invention include various aromatic compounds such as the low and high molecular weight alkylbenzenes, including low molecular weight alkylbenzenes such as toluene and the isomeric xylenes and mixtures of such materials. Higher molecular weight alkylbenzenes, typically with a molecular weight of 300 to 3,000, may be alkylated in this way as well as polynuclear aromatics including anthracene and phenanthrene. The process is, however, of primary applicability with the production of alkylated naphthalenes since these products have been found to provide lubricant materials of very good stability which may be blended with other lubricant components such as the poly-alphaolefins. For convenience and brevity, the process is described below primarily with reference to the production of alkylated naphthalenes but it may also be used in a similar manner for the production of other alkylated aromatics.

The starting materials for the production of alkylated naphthalenes include naphthalene itself as well the substituted naphthalenes which may contain one or more short chain alkyl groups containing up to eight carbon atoms, such as methyl, ethyl or propyl. Suitable alkyl-substituted naphthalenes include alpha-methylnaphthalene, dimethylnaphthalene and ethylnaphthalene. Naphthalene itself is preferred since the resulting mono-alkylated products have better thermal and oxidative stability than the more highly alkylated materials.

Suitable alkylating agents for use in the present invention include any aliphatic or aromatic organic compound having one or more available alkylating aliphatic groups capable of alkylating the naphthalene. The alkylatable group itself should have at least 6 carbon atoms, preferably at least 8, and still more preferably at least 12 carbon atoms. For the production of functional fluids and additives, the alkyl groups on the alkyl-naphthalene preferably have from 12 to 30 carbon atoms, with particular preference to 14 to 18 carbon atoms. A preferred class of alkylating agents are olefins with the requisite number of carbon atoms, for example, the hexenes, heptenes, octenes, nonenes, decenes, undecenes, dodecenes. Mixtures of the olefins, e.g. mixtures of $C_{12}$-$C_{20}$ or $C_{14}$-$C_{18}$ olefins, are useful. Branched alkylating agents, especially oligomerized olefins such as the trimers, tetramers, and pentamers, of light olefins such as ethylene, propylene; and butylenes, are also useful. Other alkylating agents which may be used include alcohols (inclusive of monoalcohols, dialcohols, trialcohols, etc.) such as hexanols, heptanols, octanols, nonanols, decanols, undecanols and dodecanols; and alkyl halides such as hexyl chlorides, octyl chlorides, dodecyl chlorides; and higher homologs.

The alkylation reaction between the aromatic starting material and the alkylating agent is carried out in the presence of a zeolite catalyst. Where the starting material is a naphthalene, the molecular size of the alkylation products will require a relatively large pore size in the zeolite in order for the products to leave the zeolite. The use of a relatively large pore size zeolite will also tend to reduce diffusion limitations with the long chain alkylating agents. Suitable large pore size zeolites generally have pore windows defined by 12-membered oxygen ring systems and include zeolites X and Y, zeolite L, ZSM-4, ZSM-18, ZSM-20, mordenite, zeolite beta and offretite, with zeolite beta and zeolite Y, especially USY, being particularly preferred. Constraint Index, which is described in detail in U. S. Patent Nos. 4,016,218 and 4,861,932, is a well-accepted measurement of zeolite pore size and, in general the large pore size zeolites useful for the alkylation of naphthalenes by the process of the invention have a Constraint Index of not more than 2, in most cases not more than 1.

In addition, however, zeolites whose structure is that of a ten membered oxygen ring, generally regarded as the intermediate pore size zeolites may also be effective catalysts for this alkylation reaction if their structure is not too highly constrained. Thus, zeolites such as ZSM-12 (Constraint Index 2) and MCM-22 (Constraint Index 1.5) may be used. MCM-22 is described in U.S. Patent No. 4,954,325.

2

In general, it has been found that the production of alkylnaphthalenes with alpha:beta ratios of 1 and higher is favored by the use of zeolite catalysts such as zeolite beta or zeolite Y preferably USY, of controlled acidity, so as to have an alpha value below 300, preferably below 200 and, for best results, below 100, e.g., 25-50.

The alpha value of a zeolite is an approximate indication of the catalytic cracking activity of the catalyst compared to a standard catalyst. The alpha test gives the relative rate constant (rate of normal hexane conversion per volume of catalyst per unit time) of the test catalyst relative to the standard catalyst which is taken as an alpha of 1 (Rate Constant = 0.016 sec$^{-1}$). The alpha test is described in U.S. Patent 3,354,078 and in J. Catalysis, 4, 527 (1965); 6, 278 (1966); and 61, 395 (1980).

The alpha value of a zeolite may be varied by conventional techniques including variation of the silica:alumina ratio, cation exchange or by selective poisoning. In particular, the alpha value of a zeolite can be reduced by steaming. In addition, zeolites with inherently low alpha values can be producing by synthesis with trivalent elements having lower acidity than aluminum, for example boron, gallium and titanium.

In general, it has been found that lowering the alkylation temperature results in an increase in the alpha/beta ratio. Thus the process of the invention is conducted at a temperature of 50°C to 300°C and preferably 90°C to 200°C.

The zeolite employed on the process of the invention may be composited with a matrix material or binder which is resistant to the temperatures and other conditions employed in the alkylation process. Such materials include active and inactive materials and synthetic or naturally occurring zeolites as well as inorganic materials such as clays, silica and/or metal oxides such as alumina, silica or silica-alumina. The latter may be either naturally occurring or in the form of gelatinous precipitates or gels including mixtures of silica and metal oxides. Use of an active material in conjunction with the zeolite may change the conversion and/or selectivity of the catalyst. Inactive materials suitably serve as diluents to control the amount of conversion so that alkylation products can be obtained economically and orderly without employing other means for controlling the rate of reaction. Binders which may be incorporated to improve the crush strength and other physically properties of the catalyst under commercial alkylation operating conditions include naturally occurring clays, e.g., bentonite and kaolin as well as the oxides referred to above.

The relative proportions of zeolite, present in finely divided crystalline form oxide matrix may vary widely, with the crystalline zeolite content ranging from 1 to 90 percent by weight and more usually, particularly when the composite is prepared in the form of beads, in the range of 2 to 80 weight percent of the composite.

The invention will now be more particularly described with reference to the following Examples.

## Example 1

This example illustrates the effect of alkylation temperature on the alpha:beta ratio. Mono-alkylated naphthalene fluids were prepared by alkylating naphthalene with alpha $C_{14}$ olefin (1:1.2 mole ratio) in an autoclave using a fresh hydrated USY catalyst (Catalyst A) at various reactor temperatures. Table 1 compares product properties and qualities as a function of reactor temperature:

Table 1

| | Run A | Run B |
|---|---|---|
| RxR Temperature, °F (°C) | 400 (200) | 350 (180) |
| Wt% Catalyst | 5 | 10 |
| **Alkylated Naphthalene (AN) Properties:** | | |
| Pour Point, °F (°C) | <-65 (-54) | <-65 (-54) |
| KV @ 40°C, cSt | 23.85 | 22.00 |
| KV @ 100°C, cSt | 4.12 | 3.90 |
| **Composition, wt%** | | |
| Mono-Alkylated | 92 | 97 |
| Di-Alkylated | 8 | 3 |
| Alpha:Beta Ratio | 0.9 | 1.3 |
| **AN Qualities*** | | |
| B-10, % Viscosity Increase | 4.7 | 2.1 |
| Neutral Number | 0.87 | 0.49 |
| RBOT, min | 270 | 600+ |

\*    The B-10 catalytic oxidation test is described in U.S. Patents 4,994,197 and 4,952,303.  The RBOT (Rotating Bomb Oxidation Test) is in accordance with ASTM D2272.

As shown, lowering the alkylation temperature from 200°C to 180°C (400 to 350°F) leads to an increase in the alpha:beta ratio from 0.9 to 1.3, resulting in a significant improvement in AN product qualities. The alpha:beta ratio of mono-alkylated naphthalene was determined by high resolution GC/MS.

**Example 2**

This example illustrates the effect of zeolite structure on the alpha:beta ratio. Alkylated naphthalenes were prepared from various zeolite catalysts including zeolite beta and USY using a similar procedure to that outlined in Example 1 (2:1 alpha $C_{14}$ = :naphthalene mole ratio). For comparison purpose, a commercial acid-treated clay (Filtrol 13) was also used to synthesize the AN product. The AN properties are compared in Table 2:

Table 2

| Catalyst | Zeolite Beta | USY | Acid-Clay |
|---|---|---|---|
| Run | 3 | 4 | 5 |
| **AN Properties** | | | |
| Pour Point, °F (C) | -55(-48) | -40(-40) | -45(-43) |
| KV @ 40°C, cSt | 65.69 | 58.92 | 93.83 |
| KV @ 100°C, cSt | 8.79 | 8.54 | 11.02 |

Table 3 shows the alkylation isomer distribution of alkylated naphthalene products:

Table 3

| Substitution Position on | | Beta | USY | Acid-Clay |
|---|---|---|---|---|
| Naphthalene | Alkyl Group | | | |
| B | 2 | 14 | 15 | 29 |
| A | 2 | 23 | 15 | 5 |
| B | 3 | 7 | 7 | 13 |
| A | 3 | 12 | 12 | 4 |
| B | 4 | 4 | 5 | 10 |
| B | 5 | 4 | 5 | 10 |
| A | 5 | 7 | 6 | 2 |
| A | 6 | 6 | 5 | 2 |
| A + B | 1 + 4 | 14 | 16 | 12 |
| B | 7 | 0 | 0 | 11 |
| A | 7 | 10 | 4 | 3 |
| Total Alpha:Beta Ratio | | 1.7 | 1.6 | 0.2 |

The results indicate that the zeolite catalysts, zeolite beta and USY, promote the formation of alpha-over beta-substitution compared to an acid-treated clay catalyst.

**Example 3**

This example illustrates the effect of zeolite acidity on the formation of alpha- and beta-position isomers of AN products. Alkylated naphthalene fluids were prepared from zeolite beta at various acidity levels. Catalyst B is a fresh zeolite beta containing 35 wt% alumina binder, having an acidity of 220 alpha. The Catalyst B was then steamed to reduce the acidity from 220 to 56 (Catalyst C) and finally to 36 alpha (Catalyst D). Alkylation reactions were carried out similar to that used in Example A using 2:1 molar ratio of alpha $C_{14}$ =:naphthalene at 180°C (350°F). Table 4 compares AN properties and compositions:

Table 4

| Catalyst | B | C | D |
|---|---|---|---|
| Catalyst ALPHA | 220 | 56 | 36 |
| AN Properties: | | | |
| Pour Point, °F (°C) | -50(-46) | 55(-48) | -55(-48) |
| KV @ 40°C, cSt | 18.55 | 29.80 | 49.54 |
| KV @ 100°C, cSt | 3.90 | 5.21 | 7.26 |
| Alpha:Beta Ratio | 0.83 | 1.36 | 1.67 |

The results indicate that decreasing zeolite acidity by steaming significantly increases the alpha:beta ratio. The ratio increases two fold from 0.83 to 1:67 when zeolite beta acidity is reduced from 220 to 36 alpha.

In addition to zeolite beta, the positive effect of steaming on the alpha:beta ratio is also applicable to other zeolite catalyst systems, such as USY. Table 5 shows that a reduction in USY acidity by steaming leads to a significant increase in the alpha:ratio from about 1.3 to about 1.6:

Table 5

| Catalyst | Fresh USY | Steamed USY |
|---|---|---|
| AN Properties: | | |
| Pour Point, °F (°C) | <-65(-54) | -50(-46) |
| KV @ 40°C, cSt | 22.92 | 50.47 |
| Alpha:Beta Ratio | 1.26 | 1.58 |

## Claims

1. A process for preparing an alkyl-substituted aromatic compound comprising reacting an aromatic compound with an alkylating agent possessing an alkylating aliphatic group having at least six carbon atoms at a temperature of 50°C to 300°C in the presence of an alkylation catalyst comprising a porous crystalline zeolite having an alpha value of less than 300 to form an alkylated aromatic compound possessing at least one alkyl group derived from the alkylating agent.

2. The process according to Claim 1 in which the alkylation temperature is from 90°C to 200°C.

3. The process according to Claim 1 or Claim 2 in which the zeolite has an alpha value less than 100.

4. The process according to any preceding Claim in which the zeolite has a Constraint Index no greater than 2.

5. The process according to any preceding Claim in which the zeolite is selected from zeolite beta, zeolite Y and zeolite USY.

6. The process according to any preceding Claim in which the alkylating aliphatic group contains 12 to 30 carbon atoms.

7. The process according to any preceding Claim in which the alkylating agent comprises an olefin.

8. The process according to any preceding Claim in which the aromatic compound comprises naphthalene or a substituted naphthalene.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-5 034 563 (ASHJIAN ET AL) * claims * | 1-8 | C07C2/66 |

-----

TECHNICAL FIELDS SEARCHED (Int. Cl.5)

C07C

The present search report has been drawn up for all claims

| Place of search | Date of completion of the search | Examiner |
|---|---|---|
| THE HAGUE | 08 JUNE 1993 | J. VAN GEYT |

EPO FORM 1503 03.82 (P0401)